(19) Europäisches Patentamt
European Patent Office
Office européen des brevets

(11) Publication number: **0 145 078 B1**

(12) **EUROPEAN PATENT SPECIFICATION**

(45) Date of publication of patent specification: **12.10.88**

(51) Int. Cl.$^4$: **C 07 D 307/87,** C 07 D 209/48, C 07 C 79/35, A 01 N 43/12

(21) Application number: **84201740.2**

(22) Date of filing: **26.11.84**

# (54) Diphenyl ether herbicides.

(30) Priority: **02.12.83 GB 8332268**
**11.05.84 GB 8412061**

(43) Date of publication of application:
**19.06.85 Bulletin 85/25**

(45) Publication of the grant of the patent:
**12.10.88 Bulletin 88/41**

(84) Designated Contracting States:
**AT BE CH DE FR GB IT LI LU NL SE**

(56) References cited:
**EP-A-0 184 256**
**US-A-4 334 915**

**JOURNAL OF ORGANIC CHEMISTRY, vol. 45, no. 8, 11th April 1980, pages 1374-1379, American Chemical Society, Easton, US; H.M. RELLES et al.: "Diaryl ether formation via nitro displacement with 4-methylphenol and potassium carbonate on 4-nitro-N-methylphthalimide"**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Haddock, Ernest**
**187 Queensborough Road**
**Sheerness Sheppey Kent (GB)**
Inventor: **Clark, Michael Thomas**
**Torcross Wises Lane**
**Borden Sittingbourne Kent (GB)**
Inventor: **Gilmore, Ian James**
**32 Waterloo Road**
**Sittingbourne Kent (GB)**

(74) Representative: **Hunter, Keith Roger Ian et al**
**4 York Road**
**London SE1 7NA (GB)**

**EP 0 145 078 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:

JOURNAL OF ORGANIC CHEMISTRY, vol. 42, no. 21, 14th October 1977, pages 3414-3419, American Chemical Society, Easton, US; F.J. WILLIAMS et al.: "Reactions of phenoxides with nitro- and halo-substituted phthalimides"

JOURNAL OF ORGANIC CHEMISTRY, vol. 42, no. 21, 14th October 1977, pages 3425-3431, American Chemical Society, Easton, US; F.J. WILLIAMS et al.: "A direct synthesis of phenoxy-substituted phthalic anhydrides by aromatic nucleophilic displacement"

CHEMICAL ABSTRACTS, vol. 73, no. 19, 9th November 1970, page 349, no. 98664z, Columbus, Ohio, US; V. OZOLINA et al.: "New analogs of 2-aryl-1,3-indandiones in the diphenyl oxide series"

CHEMICAL ABSTRACTS, vol. 80, no. 11, 18th March 1974, page 341, no. 59757g, Columbus, Ohio, US; M. LACOVA: "Phthalides and 1,3-indandiones. LIII. Phthalides and 1,3-indandiones from (arylthio)acetic and aryloxyacetic acids" & CHEM. ZVESTI 1973, 27(4), 525-35

**Description**

This invention relates to a composition for and a method of controlling undesired plant growth and to compounds for use in such a composition or method.

Certain substituted phenoxy phthalates are disclosed as herbicides in US Patent 4334915, along with the corresponding phenoxy phthalic anhydride. That anhydride is disclosed only as being a useful intermediate in the preparation of the phthalates and no mention or suggestion is made that the anhydride itself may exhibit any biological activity. It has now been found that such phenoxy phthalic anhydrides, along with certain other diphenyl ethers which bear a fused ring substituent at the 3,4 position, show interesting herbicidal activity.

Certain phenoxy phthalic acid derivatives have been disclosed in the literature, ie, J. Org. Chem., Vol. 42, 1977, pages 3414 to 3419 and 3425 to 3431 and Vol. 45 (1980), pages 1374 to 1379, Chem. Abstracts, 70, 98664z and US Patent No. 4334915 although no mention is made of herbicidal activity of these compounds.

Accordingly, the present invention provides a herbicidal composition which comprises a liquid carrier comprising a surface active agent or a solid carrier and, as active ingredient, a diphenyl ether derivative having the general formula I:

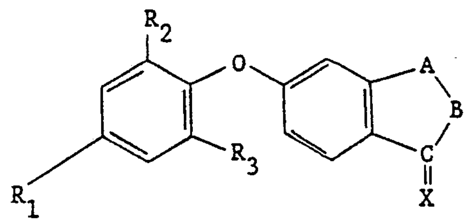

(I)

wherein

$R_1$ represents a hydrogen or halogen, preferably chlorine, atom or an alkyl or haloalkyl, preferably trifluoromethyl, group;

$R_2$ and $R_3$, which may be the same or different, each independently represents a hydrogen or halogen, preferably chlorine, atom or an alkyl, haloalkyl, nitro or cyano group;

A represents a divalent group selected from —X—,

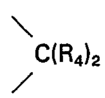 and 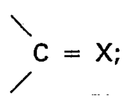

B represents a divalent group selected from

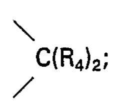 —X—; 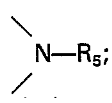 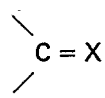 and 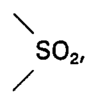

provided that when A represents —X— then B must be different from A and cannot represent

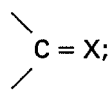

each $R_4$, which may be the same or different, represents a hydrogen or halogen atom or an optionally substituted alkyl, cycloalkyl, alkenyl, alkynyl, aryl, suitably phenyl, aralkyl, alkaryl, alkoxy, cycloalkoxy, alkenyloxy, alkynyloxy, alkoxycarbonylalkoxy, alkylthio, acyl, acyloxy, carboxyl, alkoxycarbonyl or heterocyclic group, amino group of formula $NR_6R_7$, or when one $R_4$ represents a hydrogen atom, an alkoxy group or a hydrocarbon group, then the other may represent a hydroxyl group, or both groups $R_4$ together may represent an imino group of formula $= NR_6$;

$R_5$ represents a hydrogen atom, a carboxyl or hydroxyl group, or an optionally substituted alkyl, alkoxy, aralkyl, alkoxycarbonyl or alkoxycarbonylalkyl group, or an amino group of formula $NR_6R_7$;

$R_6$ and $R_7$, which may be the same or different, each represents a hydrogen atom or an optionally substituted alkyl, aryl or acyl group;

and X, both in formula I and in the definitions of the substituents therein, represents an oxygen or sulphur atom.

The term "acyl" is used herein to denote the radical derived from an organic acid by the removal of a hydroxyl group; the organic acid may be a carboxylic acid (including carbamic acid derivatives) or a sulphonic acid, examples of suitable acyl groups being alkylcarbonyl, alkylcarbamoyl and alkylsulphonyl, e.g. acetyl, methylsulphonyl and dimethylcarbamoyl.

When any of the foregoing substituents represents or contains an alkyl, alkenyl or alkynyl substituent group, this may be linear or branched and may contain up to 10, preferably up to 6, carbon atoms, suitable

examples being methyl, ethyl, propyl, allyl and propynyl. When they represent or contain a cycloalkyl substituent group this may contain from 3 to 10, preferably 5 to 8, carbon atoms, and is suitably cyclohexyl. When they contain a haloalkyl substituent group, this suitably contains up to 6, preferably up to 4, carbon atoms and the halogen atom is suitably fluorine or chlorine, trifluoromethyl being particularly preferred. When they contain an aryl substituent group, this is preferably a phenyl group. When they contain a heterocyclic group, this may, for example, be a pyrrole, pyrrolidine, pyridine, piperidine, furan or pyran ring. When any of the foregoing substituents are designated as being optionally substituted, the substituent groups which are optionally present may be any of those customarily employed in the development of pesticidal compounds, and/or the modification of such compounds to influence their structure/activity, persistence, penetration or other property. Specific examples of such substituents include halogen, especially chlorine, atoms and nitro, cyano, hydroxyl, alkoxy, e.g. methoxy, or ester, e.g. alkoxycarbonyl, groups.

Preferred compositions are those wherein the active ingredient is a compound of general formula I wherein X represents an oxygen atom;

A represents an oxygen atom, a carbonyl group, the group $C(R_4)_2$ wherein one group $R_4$ is a hydrogen atom, or an alkyl or alkoxy group of up to 4 carbon atoms, especially methyl or methoxy, or an aryl, especially phenyl, group, and the other group $R_4$ is a hydrogen or halogen, especially chlorine or bromine, atom, a hydroxyl group, an alkyl group of up to 4 carbon atoms, especially methyl, an alkoxy group of up to 4 carbon atoms, especially methoxy, ethoxy or propoxy, optionally substituted by a halogen, especially chlorine, atom, or a hydroxyl or alkoxy, especially methoxy, group, a cycloalkoxy group of 5 to 8 carbon atoms especially cyclohexyloxy, an alkenyloxy or alkynyloxy group of up to 6 carbon atoms, especially allyloxy or propynyloxy, an alkoxycarbonylalkoxy, especially ethoxycarbonylethoxy, group, an alkylthio group of up to 4 carbon atoms, especially methyl- or propyl- thio, an alkanoyl, especially acetyl, group, a pyridyl group, or an amino group of formula $NR_6R_7$ wherein $R_6$ represents a hydrogen atom or an alkyl group of up to 4 carbon atoms, especially methyl, and $R_7$ represents an alkyl group of up to 4 carbon atoms, especially methyl, a phenyl group optionally substituted by at least one halogen, especially chlorine, atom, or an alkylcarbonyl, alkylcarbamoyl or alkylsulphonyl group, especially acetyl, dimethylcarbamoyl or methylsulphonyl;

and B represents an oxygen atom, the group

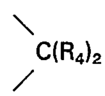

wherein each group $R_4$, which may be the same or different, independently represents a hydrogen atom or an alkyl or alkoxy group of up to 4 carbon atoms, especially methyl or methoxy, or the group $NR_5$ wherein $R_5$ represents a hydrogen atom, an alkyl group of up to 4 carbon atoms, especially methyl, a benzyl group, or an alkoxycarbonylalkyl group of up to 8 carbon atoms, especially ethoxycarbonylmethyl or ethoxycarbonylethyl.

Particularly preferred compositions are those wherein the active ingredient is a compound in which $R_1$ represents a trifluoromethyl group, $R_2$ represents a halogen, especially chlorine, atom and $R_3$ represents a hydrogen atom.

With certain exceptions, the compounds of formula I are novel, and the invention therefore also extends to those novel compounds *per se.* The novel compounds are the diphenyl ether derivatives of formula I wherein the substituents A, B, $R_1$, $R_2$, $R_3$, $R_4$, $R_5$, $R_6$ and $R_7$ have the meanings defined above, with the exception of 4-(2-chloro-4-trifluoromethylphenoxy)-phthalic anhydride and compounds where $R_1$ is a hydrogen atom or a methyl group, $R_2$ and $R_3$ each represent a hydrogen atom, A represents

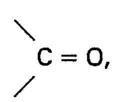

X represents an oxygen atom and B represents $NR_5$ (where $R_5$ is methyl or phenyl),

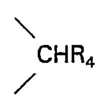

(where $R_4$ is phenyl) or —O—.

It will be appreciated that when the nature of the substituents is such as to introduce an asymmetric carbon atom, then the resulting compound will exist in stereoisomeric forms. Also, some substituent combinations permit the existence of tautomeric forms. The scope of the present invention includes these different forms of the compounds and their mixtures, and herbicidal compositions containing the active ingredient in such isomeric forms and mixtures.

The invention further provides a process for the preparation of the novel diphenyl ethers as defined above which comprise reacting a phthalide of formula:

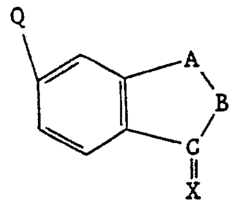

(II)

with a compound of the formula:

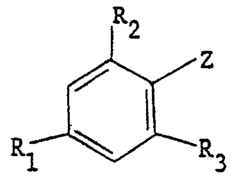

(III)

wherein A, B, X, $R_1$, $R_2$ and $R_3$ have the meanings given above for formula I; one of Z and Q represents a halogen, suitably chlorine, atom or nitro group, and the other represents a group of formula —OM wherein M represents a hydrogen or alkali metal atom. Conveniently the reaction is carried out by reacting a compound of formula II wherein Q is hydroxy with an alkali metal hydroxide in an alkanol, e.g. ethanol, solvent, thereby forming the corresponding alkali metal salt which is then reacted with a compound of formula III wherein Z represents a chlorine atom. The reaction of the said salt with the chloro- compound is preferably carried out in a suitable organic solvent, e.g. dimethyl sulphoxide, sulpholane, dimethyl formamide, or dimethyl acetamide at elevated temperature, e.g. above 25°C and in particular between 40°C and reflux, conveniently under reflux, and also under an inert atmosphere such as nitrogen.

Compounds wherein X in formula I represents sulphur may be obtained from the corresponding oxygen analogues by reaction with phosphorus pentasulphide. Compounds wherein A represents a halomethylene group may suitably be obtained by reacting the corresponding methylene compound with an appropriate halogenating agent. Thus, a compound wherein A represents CHBr may be obtained by reacting the corresponding

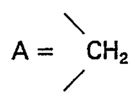

compound with N-bromosuccinimide, suitably in the presence of a peroxide such as benzoyl peroxide and in a solvent such as carbon tetrachloride. Compounds wherein A represents a hydroxymethylene group may be obtained by hydrolysis of the corresponding halomethylene compound.

Compounds wherein X and B in formula I both represent an oxygen atom, and A is the group $C(R_4)_2$ in which one group $R_4$ is a hydrogen atom and the other has one of the meanings defined above excluding hydrogen or hydroxyl, may be obtained from the corresponding compound wherein A represents CH(OH) by reaction with a reagent of formula $HR_4$ wherein $R_4$ is as defined for formula I excluding hydrogen or hydroxyl or, when $R_4$ is a carboxylic acyloxy group, an acyl anhydride of formula $Ac_2O$ wherein Ac is an alkylcarbonyl group. The reaction is suitably carried out by refluxing the reaction mixture. In some cases the reactant $HR_4$ can be used in excess to provide a convenient reaction solvent; in other cases it may be convenient to introduce the reagent $HR_4$ as a solution in an organic solvent, e.g. acetonitrile or methyl ethyl ketone.

The compounds of the general formula I have been found to show interesting activity as herbicides. Accordingly the invention also provides the use as a herbicide of such a compound or composition according to the invention. Further in accordance with the invention there is provided a method of combating undesired plant growth at a locus by treating the locus with a compound or composition according to the invention. Application to the locus may be pre-emergence or post-emergence. The dosage of active ingredient used may, for example, be from 0.05 to 4 kg/ha.

A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating herbicidal compositions may be used. Preferably compositions according to the invention contain 0.5 to 95% by weight of active ingredient.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montomorillonites and micas; calcium carbonate; calcium sulphate; ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloro-ethane. Mixtures of different liquids are often suitable.

Agricultural compositions are often formulated and transported in a concentrated form which is subsequently diluted by the user before application. The presence of small amounts of a carrier which is a surface-active agent facilitates this process of dilution. Thus preferably at least one carrier in a composition according to the invention is a surface-active agent. For example the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohol or alkyl phenols, for example *p*-octylphenol or *p*-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 or 75% w of active ingredient and usually contain in addition to solid inert carrier, 3—10% w of a dispersing agent and, where necessary, 0—10% w of stabiliser(s) and/or other additives such as penetrants or stickers. Dusts are usually formulated as a dust concentrate having a similar composition to that of a wettable powder but without a dispersant, and are diluted in the field with further solid carrier to give a composition usually containing ½—10% w of active ingredient. Granules are usually prepared to have a size between 10 and 100 BS mesh (1.676—0.152 mm), and may be manufactured by agglomeration or impregnation techniques. Generally, granules will contain ½—75% w active ingredient and 0—10% w of additives such as stabilisers, surfactants, slow release modifiers and binding agents. The so-called "dry flowable powders" consist of relatively small granules having a relatively high concentration of active ingredient. Emulsifiable concentrates usually contain, in addition to a solvent and, when necessary, co-solvent, 10—50% w/v active ingredient, 2—20% w/v emulsifiers and 0—20% w/v of other additives such as stabilisers, penetrants and corrosion inhibitors. Suspension concentrates are usually compounded so as to obtain a stable, non-sedimenting flowable product and usually contain 10—75% w active ingredient, 0.5—15% w of dispersing agents, 0.1—10% w of suspending agents such as protective colloids and thixotropic agents, 0—10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope of the invention. The said emulsions may be of the water-in-oil or of the oil-in-water type, and may have a thick 'mayonnaise'-like consistency.

The composition of the invention may also contain other ingredients, for example other compounds possessing herbicidal, insecticidal or fungicidal properties.

The following Examples illustrate the invention.

### Example 1

5-(2'-chloro-4'-trifluoromethyl phenoxy)phthalide

5-Hydroxy phthalide (5.5 g) was dissolved in ethanol (50 ml), 2.5 g potassium hydroxide added and the solution refluxed for 3 hours. The solvent was removed, the residue dissolved in dimethylsulphoxide (50 ml) and 8.2 g of 3,4 dichlorobenzotrifluoride added. This mixture was stirred under dry nitrogen at 160°C for 18 hours, after which it was cooled, poured onto ice, neutralised with dilute hydrochloric acid and the product extracted with methylene chloride to yield the desired product m.pt. 109—111°C.

| | | |
|---|---|---|
| Analysis: Calc. for $C_{15}H_8O_3ClF_3$: | C 54.8; | H 2.4% |
| Found: | C 54.8; | H 2.5% |

### Example 2

5-(2'-Chloro-4'-trifluoromethylphenoxy)-3-bromo-phthalide

The product of Ex. 1 (1.5 g) and N-bromosuccinimide (0.82 g) was dissolved in dry carbon tetrachloride (25 ml) and a small pellet of benzoyl peroxide added. The reaction mixture was refluxed with stirring under strong light, when succinimide began to precipitate. The mixture was cooled, filtered, washed with carbon

tetrachloride and the solvent removed. Chromatographed separation (silica column; methylene chloride eluant) yielded a pale orange viscous oil as the desired product.

Analysis: Calc. for $C_{15}H_7ClBrF_3O_3$: C 44.2; H 1.7%
Found: C 44.2; H 1.9%

Example 3

5-(2′-Chloro-4′-trifluoromethylphenoxy)-1-thiophthalide

The product of Example 1 (1.55 g) and phosphorus pentasulphide (2.2 g) were refluxed in sodium dried dioxan (15 ml) for 1 hour with stirring. The reaction mixture was cooled, filtered, dried, washed with methylene chloride, and chromatographically purified to yield a pale yellow solid, m.pt. 86—88°C.

Analysis: Calc. for $C_{15}H_8ClO_2SF_3$: C 52.2; H 2.3
Found: C 51.8; H 2.3

Example 4

N-Benzyl-S-(2′-nitro-4′-trifluoromethylphenoxy)phthalimide

N-Benzyl-5-hydroxyphthalimide (0.84 g) and potassium hydroxide (0.19 g) were dissolved in ethanol (50 ml) and the solvent was evaporated. The residue was dissolved in dimethylsulphoxide (50 ml) and 4-chloro-3-nitro benzotrifluoride (0.75 g) was added. The mixture was heated at 180°C for six hours and poured into water. The mixture was extracted with ethyl acetate, the ethyl acetate layer was separated, dried and evaporated to yield a pale yellow oil. The oil crystallised from ethanol as pale yellow prisms m.pt. 166—167°C.

Analysis: Calc. for $C_{22}H_{13}F_3N_2O_5$: C 59.7; H 2.9: N 6.3
Found: C 59.8; H 3.0; N 6.6

Example 5

N-Benzyl-5-(2′-chloro-4′-trifluoromethylphenoxy)phthalimide

Following a similar procedure to that of Example 4, but using 3,4-dichlorobenzotrifluoride in place of 4-chloro-3-nitrobenzotrifluoride, the desired product was obtained as a white solid m.pt. 119—121°C.

Analysis: Calc. for $C_{22}H_{13}F_3ClNO_3$: C 61.2; H 3.0: N 3.25%
Found: C 61.2; H 2.9; N 3.1%

Example 6

5-(2′-chloro-4′-trifluoromethylphenoxy)-3-hydroxyphthalide

The product of Example 2, was refluxed with 20 ml water for 3 days, and the reaction mixture extracted with methylene chloride and dried. Removal of the solvent and chromatographic purification yielded the desired product, m.pt. 145—147°C.

Analysis: Calc. for $C_{15}H_8O_4ClF_3$: C 52.2; H 2.3%
Found: C 51.9; H 2.4%

Example 7

5-(2′-chloro-4′-trifluoromethylphenoxy)-3-methoxyphthalide

A) 5-Hydroxy phthalide (5.5 g) was dissolved in ethanol (50 ml), 2.5 g potassium hydroxide added and the solution refluxed for 3 hours. The solvent was removed, the residue dissolved in dimethylsulphoxide (50 ml) and 8.2 g of 3,4-dichlorobenzotrifluoride added. This mixture was stirred under dry nitrogen at 160°C for 18 hours, after which it was cooled, poured onto ice, neutralised with dilute hydrochloric acid and the product extracted with methylene chloride.

B) The product of A (1.5 g) and N-bromosuccinimide (0.82 g) was dissolved in dry carbon tetrachloride (25 ml) and a small pellet of benzoyl peroxide added. The reaction mixture was refluxed with stirring under strong light, when succinimide began to precipitate. The mixture was cooled, filtered, washed with carbon tetrachloride and the solvent removed. Chromatographic separation (silica column; methylene chloride eluant) yielded a pale orange viscous oil.

C) The product of B was refluxed with 20 ml water for 3 days, and the reaction mixture extracted with methylene chloride and dried. Removal of the solvent and chromatographic purification yielded 5-(2′-chloro-4′-trifluoromethylphenoxy)-3-hydroxyphthalide m.pt. 145—147°C. This product is referred to in the following Examples 8—10 as "hydroxy-phthalide".

D) Hydroxyphthalide prepared as in steps A)—C) above (1.4 g) was refluxed in methanol (20 ml) with stirring for 2 hours. Residual solvent was removed by evaporation, the product chromatographically purified over silica with methylene dichloride eluant to yield a clear oil, which was recrystallised to give the desired product, m.pt. 67°C.

Analysis: Calc. for $C_{16}H_{10}ClF_3O_4$: C 53.6; H 2.8; Cl 9.9%
Found: C 53.6; H 2.9; Cl 9.9%

Example 8

5-(2′-chloro-4′-trifluoromethylphenoxy)-3-acetoxy phthalide

Hydroxyphthalide, (0.7 g) was refluxed in acetic anhydride (10 ml) with stirring for 5 hours. Excess

acetic anhydride was removed by evaporation, the residue washed with saturated sodium bicarbonate solution, extracted with methylene dichloride and the organic layers washed with bicarbonate and dried with sodium sulphate. The solvent was removed, the product chromatographically purified and recrystallised to yield a solid, m.pt. 113°C.

| | | |
|---|---|---|
| Analysis: Calc. for $C_{17}H_{10}ClF_3O_5$: | C 52.8; | H 2.6% |
| Found: | C 53.0; | H 2.7% |

Example 9

5-(2'-chloro-4'-trifluoromethylphenoxy)-3-N',N'-dimethylureido phthalide

Hydroxyphthalide (0.7 g) was added to a solution of 1,1-dimethyl urea (0.2 g) in methyl ethyl ketone (20 ml) and the mixture refluxed for 2 hours, after which a further 0.2 g of the urea were added. After a further 6 hours reflux, the solvent was removed, the residue suspended in water and shaken to remove any unreacted urea, taken up in ethyl acetate and chromatographically purified to yield a solid, m.pt. 154°C.

| | | | |
|---|---|---|---|
| Analysis: Calc. for $C_{18}H_{14}N_2O_4ClF_3$: | C 52.7; | H 3.4; | N 6.8% |
| Found: | C 52.0; | H 3.2; | N 6.4% |

Example 10

5-(2'-chloro-4'-trifluoromethylphenoxy)-3-3,4-dichloroanilino phthalide

Hydroxyphthalide (1.0 g) was added to a solution of 3,4-dichloroaniline (0.5 g) in dry acetonitrile (10 ml), and a crystal of p-toluene sulphonic acid added. The mixture was refluxed with stirring for 55 hours, the solvent removed, and the residue chromatographically purified to yield a solid, m.pt. 168°C.

| | | | |
|---|---|---|---|
| Analysis: Calc. for $C_{21}H_{11}NO_3Cl_3F_3$: | C 51.6; | H 2.2; | N 2.9 |
| Found: | C 51.6; | H 2.1; | N 2.7 |

Examples 11—40

Following procedures similar to those described in the foregoing Examples, further compounds were prepared whose characteristics and analyses are set out in Table 1. In all cases, the identity of the product was confirmed by n.m.r. analysis. In those instances marked "m.m.s." the empirical formula was confirmed by accurate mass measurement spectrometry. The compounds are identified by reference to substituent $R_2$, A and B in the following formula:

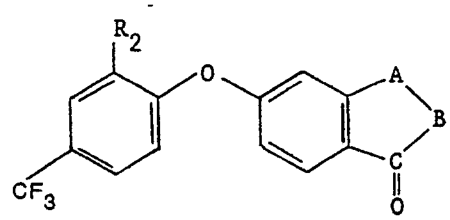

Table 1

| Example No. | A | B | $R_2$ | m.pt. °C | Analysis | | | |
|---|---|---|---|---|---|---|---|---|
| 11 | $CH_2$ | O | $NO_2$ | 104-105 | Calc. | C 53.1 | H 2.4 | N 4.1% |
| | | | | | Found | C 53.0 | H 2.4 | N 4.1% |
| 12 | $CH_2$ | O | CN | 109-111 | Calc. | C 60.2 | H 2.5 | N 4.4% |
| | | | | | Found | C 59.5 | H 2.4 | N 4.0% |
| 13 | $CH_2$ | N-Benzyl | Cl | Yellow Oil | Calc. | C 63.2 | H 3.6 | N 3.4% |
| | | | | | Found | C 63.3 | H 3.5 | N 3.7% |
| 14 | CO | O | Cl | 74-75 | Calc. | C 52.6 | H 1.7% | |
| | | | | | Found | C 52.3 | H 1.3% | |
| 15 | $CHSC_3H_7$ | O | Cl | 85 | Calc. | C 53.7 | H 3.5 | |
| | | | | | Found | C 53.5 | H 3.4 | |
| 16 | $CHOC_2H_5$ | O | Cl | Oil | Calc. | C 54.8 | H 3.2 | |
| | | | | | Found | C 54.9 | H 3.2 | |
| 17 | $CHOC_3H_7$ | O | Cl | Oil | Calc. | C 55.9 | H 3.6 | |
| | | | | | Found | C 56.0 | H 3.7 | |
| 18 | $CHOC_3H_7$ | O | Cl | Oil | Calc. | C 55.9 | H 3.6 | |
| | | | | | Found | C 55.9 | H 3.8 | |
| 19 | $CHOCH_2CH{=}CH_2$ | O | Cl | Oil | Calc. | C 56.2 | H 3.1 | |
| | | | | | Found | C 56.3 | H 3.3 | |

Table 1 continued...

| Example No. | A | B | $R_2$ | m.pt.°C | Analysis | | | |
|---|---|---|---|---|---|---|---|---|
| 20 | $CHOCH_2C\equiv CH$ | O | Cl | Oil | Calc.<br>Found | C 56.4<br>C 56.7 | H 2.6<br>H 2.8 | |
| 21 | $CHOC_6H_{11}$ | O | Cl | 99-100 | Calc.<br>Found | C 59.1<br>C 58.9 | H 4.2<br>H 4.1 | |
| 22 | $CHNHSO_2CH_3$ | O | Cl | 234-238 | Calc.<br>Found | C 45.6<br>C 45.1 | H 2.6<br>H 2.9 | N 3.3<br>N 3.4 |
| 23 | $CHNHCOCH_3$ | O | Cl | 234-6 | Calc.<br>Found | C 52.9<br>C 52.4 | H 2.9<br>H 2.7 | N 3.6<br>N 3.5 |
| 24 | $CHOCH(CH_3)$-<br>$COOC_2H_5$<br>(Isomer 2) | O | Cl | Oil | Calc.<br>Found | C 54.8<br>C 54.2 | H 3.2<br>H 3.2 | |
| 25 | $CHSCH_3$ | O | Cl | 95-98 | Calc.<br>Found | C 51.3<br>C 50.8 | H 2.7<br>H 2.8 | |
| 26 | $CHO(CH_2)_2$-<br>$OCH_3$ | O | Cl | 95-6 | Calc.<br>Found | C 53.7<br>C 53.8 | H 3.5<br>H 3.5 | |
| 27 | $CHO(CH_2)_2Cl$ | O | Cl | Oil | Calc.<br>Found | C 50.1<br>C 50.0 | H 2.7<br>H 2.8 | |

Table 1 continued...

| Example No. | A | B | $R_2$ | m.pt.°C | Analysis | | | |
|---|---|---|---|---|---|---|---|---|
| 28 | CO | NH | Cl | 183-5 | Calc.<br>Found | C 52.7<br>C 52.5 | H 2.0<br>H 1.9 | N 4.1<br>N 4.0 |
| 29 | CO | $N-CH_3$ | Cl | 110-2 | Calc.<br>Found | C 54.0<br>C 53.7 | H 2.5<br>H 2.3 | N 3.9<br>N 3.9 |
| 30 | CO | $N-CH_2COOEt$ | Cl | 140 | Calc.<br>Found | C 53.3<br>C 53.5 | H 3.0<br>H 2.9 | N 3.3<br>N 3.2 |
| 31 | CO | $N-CH(CH_3)COOEt$ | Cl | 114-5 | Calc.<br>Found | C 54.4<br>C 54.0 | H 3.4<br>H 3.3 | N 3.2<br>N 3.1 |
| 32 | $CH_2$ | $CH_2$ | $NO_2$ | 125 | Calc.<br>Found | C 57.0<br>C 56.8 | H 3.0<br>H 3.1 | N 4.1<br>N 4.1 |
| 33 | $CH-OCH(CH_3)-$<br>COOEt (Isomer I) | O | Cl | Oil | Calc.<br>Found | C 54.8<br>C 54.2 | H 3.2<br>H 3.2 | |
| 34 | $CH-OCH_2CH_2OH$ | O | Cl | Oil | Calc.<br>Found | C 52.5<br>C 52.9 | H 3.1<br>H 3.0 | |
| 35 | $CH-N(CH_3)_2$ | $N-CH_3$ | Cl | 82-3 | | (m.m.s.) | | |
| 36 | $CH-CH_3$ | O | Cl | Oil | | (m.m.s.) | | |

Table 1 continued...

| Example No. | A | B | $R_2$ | m.pt.°C | Analysis |
|---|---|---|---|---|---|
| 37 | CH-Pyrrolyl | O | Cl | 68-70 | Calc. C 57.9 H 2.8 N 3.6<br>Found C 57.9 H 2.8 N 3.4 |
| 38 | $C(CH_3)OCOCH_3$ | O | Cl | Oil | (m.m.s.) |
| 39 | $C(CH_3)OCH_3$ | O | Cl | Oil | (m.m.s.) |
| 40 | $C(CH_3)OH$ | O | Cl | Oil | (m.m.s.) |

Example 41

Herbicidal Activity

To evaluate their herbicidal activity, compounds according to the invention were tested using as representative range of plants: maize, *Zea mays* (Mz); rice, *Oryza sativa* (R); barnyard grass, *Echinochloa crusgalli* (BG); oat, *Avena sativa* (O); linseed, *Linum usitatissimum* (L); mustard, *Sinapsis alba* (M); sugar beet, *Beta vulgaris* (SB) and soya bean, *Glycine max* (S).

The tests fall into two categories, pre-emergence and post-emergence. The pre-emergence tests involved spraying a liquid formulation of the compound onto the soil in which the seeds of the plant specied mentioned above had recently been sown. The post-emergence tests involved two types of test, viz., soil drench and foliar spray tests. In the soil drench tests the soil in which the seedling plants of the above species were growing was drenched with a liquid formulation containing a compound of the invention, and in the foliar spray tests the seedling plants were sprayed with such a formulation.

The soil used in the tests was a prepared horticultural loam.

The formulations used in the tests were prepared from solutions of the test compounds in acetone containing 0.4% by weight of an alkylphenol/ethylene oxide condensate available under the trade mark TRITON X—155. These acetone solutions were diluted with water and the resulting formulations applied at dosage levels corresponding to 5 kg or 1 kg of active material per hectare in a volume equivalent to 600 litres per hectare in the soil spray and foliar spray test, and at a dosage of level equivalent to 10 kilograms of active material per hectare in a volume equivalent to approximately 3,000 litres per hectare in the soil drench tests.

In the pre-emergence tests untreated sown soil and in the post-emergence tests untreated soil bearing seedling plants were used as controls.

The herbicidal effects of the test compounds were assessed visually twelve days after spraying the foliage and the soil, and thirteen days after drenching the soil and were recorded on a 0—9 scale. A rating 0 indicates growth as untreated control, a rating 9 indicates death. An increase of 1 unit on the linear scale approximates to a 10% increase in the level of effect.

The results of the tests are set out in Table II below, in which the compounds are identified by reference to the preceding examples.

Table 2

| Compound of Example No. | Soil Drench 10 kg/ha | | | | | | | | Dosage kg/h | Foliar Spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | | Mz | R | BG | O | L | M | SB | S | Mz | R | BG | O | L | M | SB | S |
| 1 | 7 | 7 | 9 | 8 | 6 | 6 | 6 | 2 | 5 | 7 | 5 | 9 | 8 | 9 | 9 | 8 | 8 | 5 | 7 | 9 | 8 | 9 | 8 | 9 | 5 |
| | | | | | | | | | 1 | 3 | 4 | 9 | 8 | 9 | 8 | 9 | 8 | 0 | 4 | 9 | 7 | 8 | 7 | 9 | 4 |
| 11 | 7 | 8 | 8 | 4 | 6 | 5 | 4 | 3 | 5 | 4 | 2 | 8 | 3 | 7 | 8 | 5 | 5 | 3 | 5 | 8 | 0 | 5 | 5 | 7 | 0 |
| | | | | | | | | | 1 | 2 | 1 | 5 | 2 | 6 | 6 | 3 | 5 | 0 | 3 | 1 | 0 | 0 | 0 | 0 | 0 |
| 12 | 6 | 7 | 8 | 5 | 7 | 5 | 3 | 4 | 5 | 5 | 4 | 8 | 6 | 8 | 8 | 7 | 8 | 7 | 6 | 9 | 5 | 7 | 5 | 9 | 4 |
| | | | | | | | | | 1 | 2 | 0 | 3 | 3 | 8 | 6 | 5 | 6 | 4 | 4 | 8 | 3 | 5 | 4 | 9 | 0 |
| 2 | 3 | 5 | 5 | 6 | 2 | 3 | 4 | 0 | 5 | 7 | 5 | 9 | 8 | 9 | 9 | 9 | 9 | 7 | 7 | 8 | 6 | 6 | 6 | 8 | 0 |
| | | | | | | | | | 1 | 5 | 4 | 8 | 6 | 8 | 8 | 6 | 7 | 0 | 0 | 0 | 0 | 0 | 0 | 2 | 0 |
| 3 | 0 | 2 | 6 | 7 | 5 | 4 | 4 | 0 | 5 | 8 | 7 | 9 | 9 | 9 | 9 | 9 | 8 | 4 | 4 | 9 | 6 | 6 | 6 | 9 | 0 |
| | | | | | | | | | 1 | 8 | 6 | 8 | 8 | 9 | 8 | 9 | 8 | 2 | 4 | 9 | 5 | 5 | 5 | 9 | 0 |
| 6 | 7 | 6 | 6 | 7 | 7 | 5 | 5 | 1 | 5 | 7 | 7 | 9 | 7 | 9 | 9 | 8 | 8 | 5 | 8 | 8 | 6 | 0 | 6 | 9 | 0 |
| | | | | | | | | | 1 | 5 | 5 | 6 | 4 | 8 | 8 | 5 | 6 | 2 | 3 | 6 | 3 | 0 | 3 | 8 | 0 |
| 14 | 4 | 2 | 4 | 4 | 0 | 7 | 4 | 5 | 5 | 5 | 4 | 7 | 5 | 8 | 8 | 8 | 7 | 2 | 0 | 2 | 0 | 0 | 4 | 4 | 0 |
| | | | | | | | | | 1 | 5 | 3 | 6 | 3 | 6 | 8 | 5 | 6 | 0 | 0 | 0 | 0 | 0 | 0 | 0 | 0 |

Table 2 continued...

| Compound of Ex. No. | Soil drench 10/kg/ha | | | | | | | | Dosage kg/ha | Foliar spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | | Mz | R | BG | O | L | M | SB | S | Mz | R | BG | O | L | M | SB | S |
| 7 | 7 | 7 | 8 | 7 | 8 | 9 | 9 | 5 | 5 | 9 | 8 | 9 | 9 | 9 | 9 | 9 | 9 | 8 | 9 | 9 | 9 | 9 | 9 | 9 | 9 |
| | | | | | | | | | 1 | 8 | 8 | 9 | 9 | 9 | 9 | 9 | 9 | 7 | 8 | 9 | 8 | 9 | 9 | 9 | 9 |
| 8 | 5 | 4 | 7 | 6 | 5 | 5 | 5 | 4 | 5 | 8 | 6 | 9 | 8 | 9 | 9 | 8 | 7 | 3 | 0 | 8 | 3 | 4 | 7 | 7 | 0 |
| | | | | | | | | | 1 | 4 | 3 | 8 | 6 | 9 | 8 | 6 | 6 | 2 | 0 | 7 | 2 | 2 | 5 | 6 | 0 |
| 9 | 5 | 4 | 7 | 4 | 3 | 3 | 3 | 2 | 5 | 7 | 2 | 8 | 5 | 7 | 6 | 4 | 5 | 3 | 0 | 6 | 3 | 0 | 4 | 6 | 0 |
| | | | | | | | | | 1 | 3 | 1 | 4 | 3 | 6 | 4 | 2 | 4 | 0 | 0 | 5 | 1 | 0 | 2 | 4 | 0 |
| 10 | 4 | 4 | 7 | 5 | 3 | 3 | 2 | 3 | 5 | 6 | 4 | 8 | 6 | 8 | 8 | 6 | 6 | 0 | 0 | 6 | 0 | 3 | 4 | 5 | 0 |
| | | | | | | | | | 1 | 3 | 2 | 4 | 3 | 5 | 5 | 4 | 4 | 0 | 0 | 5 | 0 | 2 | 2 | 2 | 0 |
| 15 | 0 | 0 | 0 | 0 | 6 | 3 | 8 | 0 | 5 | 6 | 3 | 8 | 6 | 9 | 9 | 9 | 8 | 0 | 0 | 0 | 0 | 0 | 0 | 0 | 0 |
| | | | | | | | | | 1 | 5 | 2 | 8 | 5 | 9 | 9 | 9 | 8 | 0 | 0 | 0 | 0 | 0 | 0 | 0 | 0 |
| 16 | 9 | 4 | 9 | 8 | 9 | 7 | 9 | 5 | 5 | 9 | 6 | 9 | 9 | 9 | 9 | 9 | 9 | 2 | 3 | 9 | 5 | 7 | 7 | 9 | 2 |
| | | | | | | | | | 1 | 8 | 6 | 9 | 9 | 9 | 9 | 9 | 9 | 0 | 0 | 8 | 4 | 4 | 6 | 8 | 1 |

Table 2 continued....

| Compound of Ex. No. | Soil drench 10/kg/ha | | | | | | | | Dosage kg/ha | Foliar spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | | Mz | R | BG | O | L | M | SB | S | Mz | R | BG | O | L | M | SB | S |
| 17 | 4 | 3 | 7 | 7 | 6 | 3 | 6 | 0 | 5 | 8 | 7 | 9 | 9 | 9 | 9 | 9 | 9 | 4 | 3 | 8 | 6 | 7 | 8 | 9 | 5 |
| | | | | | | | | | 1 | 8 | 6 | 9 | 9 | 9 | 9 | 9 | 9 | 2 | 3 | 8 | 5 | 6 | 7 | 8 | 2 |
| 18 | 6 | 5 | 7 | 7 | 7 | 4 | 9 | 2 | 5 | 8 | 7 | 9 | 9 | 9 | 9 | 9 | 9 | 0 | 0 | 9 | 5 | 7 | 7 | 8 | 0 |
| | | | | | | | | | 1 | 7 | 6 | 9 | 9 | 9 | 9 | 9 | 8 | 0 | 0 | 7 | 3 | 6 | 6 | 5 | 0 |
| 19 | 2 | 5 | 7 | 7 | 5 | 5 | 9 | 2 | 5 | 8 | 7 | 9 | 9 | 9 | 9 | 9 | 9 | 0 | 3 | 8 | 4 | 6 | 7 | 8 | 0 |
| | | | | | | | | | 1 | 6 | 5 | 8 | 8 | 9 | 9 | 9 | 8 | 0 | 0 | 6 | 1 | 5 | 6 | 7 | 0 |
| 20 | 3 | 4 | 5 | 6 | 5 | 4 | 8 | 2 | 5 | 7 | 7 | 9 | 8 | 9 | 9 | 9 | 7 | 2 | 0 | 7 | 4 | 7 | 7 | 8 | 2 |
| | | | | | | | | | 1 | 6 | 6 | 8 | 8 | 9 | 9 | 9 | 7 | 0 | 0 | 5 | 1 | 5 | 6 | 6 | 0 |
| 21 | 0 | 0 | 2 | 3 | 3 | 2 | 8 | 0 | 5 | 5 | 5 | 6 | 5 | 8 | 9 | 8 | 8 | 3 | 4 | 0 | 0 | 6 | 6 | 8 | 0 |
| | | | | | | | | | 1 | 5 | 5 | 5 | 5 | 8 | 9 | 8 | 8 | 3 | 2 | 0 | 0 | 6 | 6 | 6 | 0 |
| 22 | 6 | 6 | 7 | 5 | 4 | 3 | 4 | 2 | 5 | 6 | 4 | 8 | 5 | 7 | 5 | 5 | 7 | 0 | 0 | 0 | 0 | 0 | 0 | 0 | 0 |
| | | | | | | | | | 1 | 4 | 2 | 4 | 3 | 5 | 4 | 4 | 4 | 0 | 0 | 0 | 0 | 0 | 0 | 0 | 0 |

Table 2 continued....

| Compound of Ex. No. | Soil drench 10/kg/ha | | | | | | | | Dosage kg/ha | Foliar spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | | Mz | R | BG | O | L | M | SB | S | Mz | R | BG | O | L | M | SB | S |
| 23 | 0 | 0 | 0 | 0 | 0 | 0 | 0 | 0 | 5 | 0 | 0 | 0 | 3 | 0 | 8 | 7 | 5 | 0 | 0 | 0 | 0 | 0 | 0 | 0 | 0 |
| | | | | | | | | | 1 | 0 | 0 | 0 | 3 | 0 | 8 | 7 | 5 | 0 | 0 | 0 | 0 | 0 | 0 | 0 | 0 |
| 25 | 0 | 5 | 4 | 6 | 5 | 4 | 9 | 0 | 5 | 7 | 6 | 8 | 9 | 9 | 9 | 9 | 8 | 0 | 0 | 4 | 0 | 0 | 6 | 7 | 0 |
| | | | | | | | | | 1 | 4 | 4 | 6 | 7 | 9 | 8 | 9 | 8 | 0 | 0 | 2 | 0 | 0 | 4 | 7 | 0 |
| 26 | 7 | 7 | 7 | 8 | 4 | 3 | 6 | 6 | 5 | 5 | 5 | 6 | 7 | 8 | 7 | 9 | 8 | 0 | 0 | 8 | 2 | 0 | 6 | 7 | 5 |
| | | | | | | | | | 1 | 5 | 4 | 6 | 7 | 8 | 7 | 8 | 6 | 0 | 0 | 5 | 1 | 0 | 3 | 7 | 0 |
| 27 | 0 | 4 | 3 | 6 | 6 | 7 | 8 | 5 | 5 | 6 | 4 | 8 | 8 | 9 | 9 | 9 | 6 | 0 | 0 | 5 | 2 | 7 | 3 | 7 | 4 |
| | | | | | | | | | 1 | 6 | 3 | 6 | 5 | 8 | 7 | 9 | 6 | 0 | 0 | 3 | 1 | 4 | 3 | 7 | 0 |
| 37 | 0 | 0 | 0 | 0 | 0 | 0 | 0 | 0 | 5 | 4 | 4 | 6 | 3 | 8 | 8 | 7 | 4 | 0 | 0 | 0 | 0 | 0 | 6 | 7 | 7 |
| | | | | | | | | | 1 | 1 | 1 | 1 | 1 | 6 | 7 | 4 | 1 | 0 | 0 | 0 | 0 | 0 | 0 | 0 | 0 |
| 34 | 6 | 7 | 7 | 8 | 6 | 0 | 4 | 5 | 5 | 8 | 6 | 8 | 7 | 9 | 8 | 7 | 8 | 4 | 4 | 8 | 3 | 8 | 8 | 9 | 4 |
| | | | | | | | | | 1 | 3 | 3 | 5 | 4 | 9 | 6 | 5 | 6 | 0 | 3 | 2 | 0 | 6 | 4 | 5 | 0 |

Table 2 continued....

| Compound of Ex. No. | Soil drench 10/kg/ha | | | | | | | | Dosage kg/ha | Foliar spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | | Mz | R | BG | O | L | M | SB | S | Mz | R | BG | O | L | M | SB | S |
| 35 | | | | | | | | | 5 | | | | | | | | | | | | | | | | |
| | | | | | | | | | 1 | 4 | 4 | 4 | 3 | 6 | 5 | 5 | 4 | 0 | 0 | 0 | 0 | 0 | 0 | 0 | 0 |
| 36 | 9 | 8 | 9 | 8 | 9 | 7 | 7 | 5 | 5 | 8 | 8 | 9 | 9 | 9 | 9 | 9 | 9 | 7 | 8 | 9 | 9 | 9 | 8 | 9 | 5 |
| | | | | | | | | | 1 | 5 | 7 | 9 | 9 | 9 | 9 | 9 | 9 | 2 | 7 | 9 | 8 | 8 | 8 | 9 | 2 |
| 28 | 0 | 0 | 2 | 0 | 0 | 3 | 3 | 4 | 5 | 5 | 5 | 6 | 4 | 5 | 5 | 6 | 4 | 0 | 0 | 0 | 0 | 0 | 0 | 5 | 0 |
| | | | | | | | | | 1 | 5 | 5 | 4 | 3 | 4 | 4 | 4 | 3 | 0 | 0 | 0 | 0 | 0 | 0 | 5 | 0 |
| 30 | 0 | 0 | 3 | 2 | 0 | 0 | 3 | 0 | 5 | 6 | 4 | 6 | 5 | 8 | 6 | 7 | 5 | 0 | 0 | 6 | 0 | 4 | 3 | 5 | 2 |
| | | | | | | | | | 1 | 4 | 2 | 4 | 4 | 8 | 5 | 5 | 3 | 0 | 0 | 3 | 0 | 2 | 1 | 5 | 1 |
| 29 | 0 | 0 | 0 | 0 | 0 | 0 | 0 | 0 | 5 | 4 | 3 | 5 | 5 | 8 | 5 | 7 | 6 | 0 | 0 | 0 | 0 | 0 | 0 | 0 | 0 |
| | | | | | | | | | 1 | 2 | 1 | 2 | 4 | 5 | 4 | 4 | 5 | 0 | 0 | 0 | 0 | 0 | 0 | 0 | 0 |
| 32 | 3 | 2 | 7 | 4 | 2 | 0 | 0 | 0 | 5 | 7 | 3 | 7 | 6 | 7 | 3 | 4 | 6 | 0 | 0 | 0 | 0 | 0 | 0 | 0 | 0 |
| | | | | | | | | | 1 | 2 | 2 | 6 | 4 | 4 | 2 | 3 | 4 | 0 | 0 | 0 | 0 | 0 | 0 | 0 | 0 |

Table 2 continued....

| Compound of Ex. No. | Soil drench 10/kg/ha | | | | | | | | Dosage kg/ha | Foliar spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | | Mz | R | BG | O | L | M | SB | S | Mz | R | BG | O | L | M | SB | S |
| 38 | 7 | 9 | 8 | 9 | 8 | 8 | 9 | 4 | 5 | 7 | 8 | 9 | 9 | 9 | 9 | 8 | 7 | 2 | 8 | 9 | 9 | 8 | 5 | 9 | 7 |
| | | | | | | | | | 1 | 3 | 8 | 9 | 9 | 9 | 8 | 6 | 6 | 0 | 6 | 8 | 8 | 6 | 4 | 8 | 0 |
| 39 | 7 | 7 | 8 | 8 | 9 | 8 | 9 | 7 | 5 | 8 | 6 | 9 | 9 | 9 | 9 | 9 | 9 | 8 | 8 | 9 | 8 | 9 | 9 | 9 | 8 |
| | | | | | | | | | 1 | 6 | 4 | 9 | 9 | 9 | 9 | 9 | 9 | 6 | 7 | 9 | 7 | 9 | 9 | 9 | 8 |

**Claims**

1. A herbicidal composition which comprises a liquid carrier comprising a surface-active agent or a solid carrier and, as active ingredient, a diphenyl ether derivative having the general formula I:

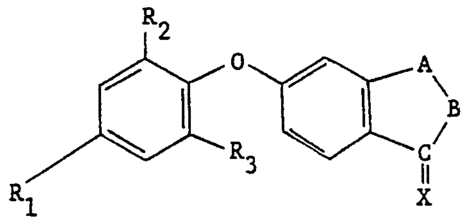

(I)

wherein $R_1$ represents a hydrogen or halogen atom or an alkyl or haloalkyl group; $R_2$ and $R_3$, which may be the same or different, each independently represents a hydrogen or halogen atom or an alkyl, haloalkyl, nitro or cyano group; A represents a divalent group selected from —X—,

$>C(R_4)_2$ and $>C = X$;

B represents a divalent group selected from

$>C(R_4)_2$; —X—; $>N—R_5$; $>C = X$ and $>SO_2$,

provided that when A represents —X—, then B must be different from A and cannot represent

$>C = X$;

each $R_4$, which may be the same or different, represents a hydrogen or halogen atom or an optionally substituted alkyl, cycloalkyl, alkenyl, alkynyl, aryl, aralkyl, alkaryl, alkoxy, cycloalkoxy, alkenyloxy, alkynyloxy, alkoxycarbonylalkoxy, alkylthio, acyl, acyloxy, carboxyl, alkoxycarbonyl or heterocyclic group, an amino group of formula $NR_6R_7$, or when one $R_4$ represents a hydrogen atom, an alkoxy group or a hydrocarbon group, then the other may represent a hydroxyl group, or both groups $R_4$ together may represent an imino group of formula $= NR_6$; $R_5$ represents a hydrogen atom, a carboxyl or hydroxyl group, or an optionally substituted alkyl, alkoxy, aralkyl, alkoxycarbonyl or alkoxycarbonylalkyl group, or an amino group of formula $NR_6R_7$; $R_6$ and $R_7$, which may be the same or different, each represents a hydrogen atom or an optionally substituted alkyl, aryl or acyl group; and X, both in formula I and in the definitions of the substituents therein, represents an oxygen or sulphur atom.

2. Compositions as claimed in claim 1 wherein the active ingredient is a compound of general formula I wherein X represents an oxygen atom; A represents an oxygen atom, a carbonyl group, the group $C(R_4)_2$ wherein one group $R_4$ is a hydrogen atom, an alkyl or alkoxy group of up to 4 carbon atoms, or an aryl group, and the other group $R_4$ is a hydrogen or halogen atom, a hydroxyl group, an alkyl group of up to 4 carbon atoms, an alkoxy group of up to 4 carbon atoms, optionally substituted by a halogen atom, or a hydroxyl or alkoxy group, a cycloalkoxy group of 5 to 8 carbon atoms, an alkenyloxy or alkynyloxy group of up to 6 carbon atoms, an alkoxycarbonylalkoxy group, an alkylthio group of up to 4 carbon atoms, an alkanoyl group, a pyridyl group, or an amino group of formula $NR_6R_7$ wherein $R_6$ represents a hydrogen atom or an alkyl group of up to 4 carbon atoms and $R_7$ represents an alkyl group of up to 4 carbon atoms, a phenyl group optionally substituted by at least one halogen atom, or an alkylcarbonyl, alkylcarbamoyl or alkylsulphonyl group; and B represents an oxygen atom, the group $C(R_4)_2$ wherein each group $R_4$, which may be the same or different, independently represents a hydrogen atom or an alkyl or alkoxy group of up to 4 carbon atoms, or the group $NR_5$ wherein $R_5$ represents a hydrogen atom, an alkyl group of up to 4 carbon atoms, a benzyl group, or an alkoxycarbonylalkyl group of up to 8 carbon atoms.

3. Compositions as claimed in claim 2 wherein B represents an oxygen atom and A represents the group $C(R_4)_2$ in which $R_4$ is as defined in claim 2.

4. Compositions as claimed in claims 1, 2 or 3 wherein $R_1$ represents a trifluoromethyl group, $R_2$ represents a halogen atom, and $R_3$ represents a hydrogen atom.

5. A composition as claimed in any one of the preceding claims which comprises at least two carriers, at least one of which is a surface-active agent.

6. A diphenyl ether derivative of the formula I as shown in claim 1, wherein the substituents have the meanings defined in claim 1, with the exception of 4-(2-chloro-4-trifluoromethylphenoxy) phthalic

anhydride and compounds where $R_1$ is a hydrogen atom or a methyl group, $R_2$ and $R_3$ each represent a hydrogen atom, A represents

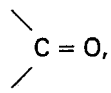

X represents an oxygen atom and B represents

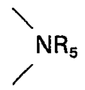

(where $R_5$ is methyl or phenyl),

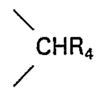

(where $R_4$ is phenyl) or —O—.

7. A diphenyl ether as claimed in claim 6 wherein the substituents of formula I have the meanings defined in any one of claims 2—4.

8. A process for the preparation of a diphenyl ether as claimed in claim 6, which comprises reacting a phthalide of formula

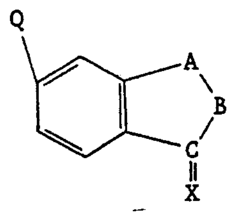

(II)

with a compound of the formula:

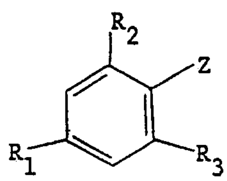

(III)

wherein A, B, $R_1$, $R_2$ and $R_3$ have the meanings defined in claim 6; and one of Z and Q represents a halogen atom or nitro group and the other represents a group of formula —OM wherein M represents a hydrogen or alkali metal atom, followed, when the thiophthalide is required, by reaction with phosphorus pentasulphide, or when the desired compound is a halo or hydroxy methylene group by reaction with a halogenating agent and, for the hydroxymethylene derivative, hydrolysis of the resultant halomethylene derivative.

9. Process as claimed in 8, wherein the initial reaction is carried out by reacting a compound of formula II wherein Q is hydroxy with an alkali metal hydroxide in an alkanol solvent, followed by reaction of the resultant alkoxide with a compound of formula III wherein Z represents a chlorine atom.

10. Process as claimed in claim 9 wherein the reaction of the alkoxide with the chloro compound III is carried out in an organic solvent at elevated temperature and under an inert atmosphere.

11. Method of combating undesired plant growth at a locus, which comprises treating the locus with a composition as claimed in any one of claims 1—5 or a compound as claimed in claim 6 or 7.

12. Use as a herbicide of a diphenyl ether as defined in any one of claims 1—7.

**Patentansprüche**

1. Herbizide Zusammensetzung, welche einen flüssigen Träger mit einem Gehalt an einem oberflächenaktiven Mittel oder einen festen Träger und als wirksamen Bestandteil ein Diphenyletherderivat mit der allgemeinen Formel I:

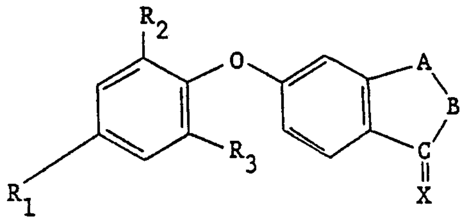

(I)

umfaßt, worin $R_1$ ein Wasserstoff- oder Halogenatom, vorzugsweise Chloratom, oder eine Alkyl- oder Halogenalkylgruppe, vorzugsweise Trifluormethylgruppe darstellt;

$R_2$ und $R_3$ die gleich oder verschieden sein können, jeweils unabhängig voneinander ein Wasserstoff- oder Halogenatom oder eine Alkyl-, Halogenalkyl-, Nitro- oder Cyanogruppe bedeuten;

A eine zweiwertige Gruppe, ausgewählt unter —X—,

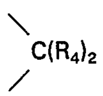 und 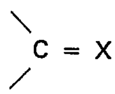

bedeutet; B eine zweiwertige Gruppe, ausgewählt unter

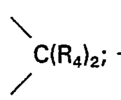 —X—; 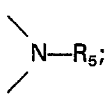 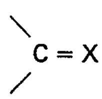 und 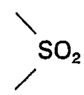

mit der Maßgabe bedeutet, daß dann, wenn A für —X— steht, B von A verschieden sein muß und nicht

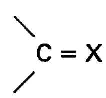

bedeuten kann;

jeder Rest $R_4$, die gleich oder verschieden sein können, ein Wasserstoff- oder Halogenatom oder eine gegebenenfalls substituierte Alkyl-, Cycloalkyl-, Alkenyl-, Akinyl-, Aryl-, Aralkyl-, Alkaryl-, Alkoxy-, Cycloalkoxy-, Alkenyloxy-, Alkinyloxy-, Alkoxycarbonylalkoxy-, Alkylthio-, Acyl-, Acyloxy-, Carboxyl-, Alkoxycarbonyl- oder heterocyklische Gruppe, eine Aminogruppe der Formel $NR_6R_7$, oder, wenn ein Rest $R_4$ ein Wasserstoffatom, eine Alkoxygruppe oder eine Kohlenwasserstoffgruppe bedeutet, der andere Rest eine Hydroxylgruppe darstellen kann, oder beide Gruppen $R_4$ zusammen eine Iminogruppe der Formel $= NR_6$ bedeuten können;

$R_5$ ein Wasserstoffatom, eine Carboxyl- oder Hydroxylgruppe oder eine gegebenenfalls substituierte Alkyl-, Alkoxy-, Aralkyl-, Alkoxycarbonyl- oder Alkoxycarbonylalkylgruppe oder eine Aminogruppe der Formel $NR_6R_7$ bedeutet;

$R_6$ und $R_7$, die gleich oder verschieden sein können, jeweils ein Wasserstoffatom oder eine gegebenenfalls substituierte Alkyl-, Aryl- oder Acylgruppe bedeuten;

und X, sowohl in Formel I als auch in den Definitionen der darin angeführten Substituenten, ein Sauerstoff- oder Schwefelatom bedeutet.

2. Zusammensetzungen nach Anspruch 1, worin der wirksame Bestandteil eine Verbindung der allgemeinen Formel I ist, worin X ein Sauerstoffatom darstellt; A ein Sauerstoffatom, eine Carbonylgruppe, die Gruppe $C(R_4)_2$, worin eine Gruppe $R_4$ ein Wasserstoffatom, eine Alkyl- oder Alkoxygruppe mit bis zu 4 Kohlenstoffatomen oder eine Arylgruppe darstellt und die andere Gruppe $R_4$ ein Wasserstoff- oder Halogenatom, eine Hydroxylgruppe, eine Alkylgruppe mit bis zu 4 Kohlenstoffatomen, eine Alkoxygruppe mit bis zu 4 Kohlenstoffatomen, die gegebenenfalls durch ein Halogenatom oder eine Hydroxyl- oder Alkoxygruppe substituiert ist, eine Cycloalkoxygruppe mit 5 bis 8 Kohlenstoffatomen, eine Alkenyloxy- oder Alkinyloxygruppe mit bis zu 6 Kohlenstoffatomen, eine Alkoxycarbonylalkoxygruppe, eine Alkylthiogruppe mit bis zu 4 Kohlenstoffatomen, eine Alkanoylgruppe, eine Pyridylgruppe oder eine Aminogruppe der Formel $NR_6R_7$, worin $R_6$ ein Wasserstoffatom oder eine Alkylgruppe mit bis zu 4 Kohlenstoffatomen darstellt und $R_7$ eine Alkylgruppe mit bis zu 4 Kohlenstoffatomen, eine gegebenenfalls durch wenigstens ein Halogenatom substituierte Phenylgruppe oder eine Alkylcarbonyl-, Alkylcarbamoyl- oder Alkylsulfonylgruppe darstellt; und B ein Sauerstoffatom, die Gruppe $C(R_4)_2$, worin jede Gruppe $R_4$, die gleich oder verschieden sein können, unabhängig voneinander ein Wasserstoffatom oder eine Alkyl- oder

Alkoxygruppe mit bis zu 4 Kohlenstoffatomen bedeutet, oder die Gruppe $NR_5$ darstellt, worin $R_5$ ein Wasserstoffatom, eine Alkylgruppe mit bis zu 4 Kohlenstoffatomen, eine Benzylgruppe oder eine Alkoxycarbonylalkylgruppe mit bis zu 8 Kohlenstoffatomen darstellt.

3. Zusammensetzung nach Anspruch 2, worin B ein Sauerstoffatom darstellt und A die Gruppe $C(R_4)_2$ bedeutet, worin $R_4$ wie in Anspruch 2 definiert ist.

4. Zusammensetzung nach Anspruch 1, 2 oder 3, worin $R_1$ eine Trifluormethylgruppe bedeutet, $R_2$ ein Halogenatom darstellt und $R_3$ ein Wasserstoffatom bedeutet.

5. Zusammensetzung nach einem der vorstehenden Ansprüche, welche wenigstens zwei Träger umfaßt, von denen wenigstens einer ein oberflächenaktives Mittel ist.

6. Diphenyletherderivat der Formel I, wie in Anspruch 1 dargestellt, worin die Substituenten die in Anspruch 1 angegebenen Bedeutungen aufweisen, mit der Ausnahme von 4-(2-Chlor-4-trifluormethylphenoxy)phthalsäureanhydrid und Verbindungen, worin $R_1$ ein Wasserstoffatom oder eine Methylgruppe ist, $R_2$ und $R_3$ jeweils ein Wasserstoffatom darstellen, A für

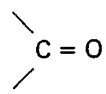

steht, X ein Sauerstoffatom darstellt und B für

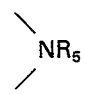

steht (worin $R_5$ Methyl oder Phenyl ist),

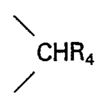

(worin $R_4$ Phenyl bedeutet) oder —O— bedeutet.

7. Diphenylether nach Anspruch 6, worin die Substituenten der Formel I die in einem der Ansprüche 2 bis 4 definierten Bedeutungen aufweisen.

8. Verfahren zur Herstellung eines Diphenylethers, wie in Anspruch 6 beansprucht, welches ein Umsetzen eines Phthalids der Formel

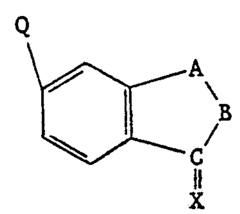

(II)

mit einer Verbindung der Formel:

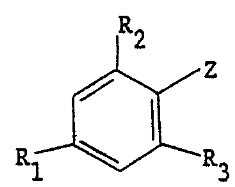

(III)

umfaßt, worin A, B, X, $R_1$, $R_2$ und $R_3$ die in Anspruch 6 angegebenen Bedeutungen besitzen; einer der Reste Z und Q ein Halogenatom oder eine Nitrogruppe bedeutet, und der andere Rest eine Gruppe der Formel —OM darstellt, worin M ein Wasserstoff- oder Alkalimetallatom bedeutet, mit anschließender Umsetzung mit Phosphorpentasulfid, wenn das Thiophthalid benötigt wird, oder, wenn die gewünschte Verbindung eine Halogen- oder Hydroxymethylengruppe aufweist, mit anschließender Umsetzung mit einem Halogenierungsmittel und, für das Hydroxymethylenderivat, Hydrolyse des erhaltenen Halogenmethylenderivats.

9. Verfahren nach Anspruch 8, worin die Anfangsreaktion durch Umsetzen einer Verbindung der Formel II, worin Q für Hydroxy steht, mit einem Alkalimetallhydroxid in einem Alkanollösungsmittel ausgeführt wird, mit anschließender Umsetzung des gebildeten Alkoxids mit einer Verbindung der Formel III, worin Z ein Chloratom bedeutet.

10. Verfahren nach Anspruch 9, worin die Umsetzung des Alkoxids mit der Chlorverbindung III in einem organischen Lösungsmittel bei erhöhter Temperatur und unter Inertatmosphäre ausgeführt wird.

11. Verfahren zur Bekämpfung von unerwünschtem Pflanzenwuchs an einem Ort, welches ein Behandeln des Ortes mit einer Zusammensetzung, wie in einem der Ansprüche 1 bis 5 beansprucht, oder mit einer Verbindung, wie in Anspruch 6 oder 7 beansprucht, umfaßt.

12. Verwendung eines Diphenylethers, wie in einem der Ansprüche 1 bis 7 definiert, als ein Herbizid.

**Revendications**

1. Composition herbicide qui comprend un véhicule liquide comprenant un agent tensio-actif ou un véhicule solide et, comme substance active, un dérivé d'éther de diphényle répondant à la formule générale I:

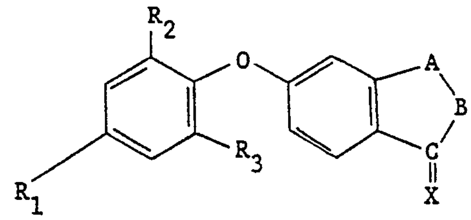

(I)

dans laquelle $R_1$ représente un atome d'hydrogène ou d'halogène ou un groupe alkyle ou halogénoalkyle; $R_2$ et $R_3$, qui peuvent être identiques ou différents, représentent chacun indépendamment un atome d'hydrogène ou d'halogène ou un groupe alkyle, halogénoalkyle, nitro ou cyano;

A représente un groupe divalent choisi parmi —X—,

$C(R_4)_2$ et $C = X$;

B représente un groupe divalent choisi parmi

$C(R_4)_2$; —X—; $N—R_5$; $C = X$ et $SO_2$,

à condition que, lorsque A représente —X—, B soit différent de A et ne représente pas

$C = X$;

Chacun des $R_4$, qui peuvent être identiques ou différents, représente un atome d'hydrogène ou d'halogène ou un groupe, éventuellement substitué, alkyle, cycloalkyle, alcényle, alcynyle, aryle, arylalkyle, alkylaryle, alcoxy, cycloalcoxy, alcényloxy, alcynyloxy, alcoxycarbonylalcoxy, alkylthio, acyle, acyloxy, carboxyle, alcoxycarbonyle ou un groupe hétérocyclique, un groupe amino de formule $NR_6R_7$ ou, lorsqu'un des $R_4$ représente un atome d'hydrogène, un groupe alcoxy ou un groupe hydrocarboné, l'autre peut alors représenter un groupe hydroxyle, ou les deux groupes $R_4$ peuvent représenter ensemble un groupe imino de formule $= NR_6$; $R_5$ représente un atome d'hydrogène, un groupe carboxyle ou hydroxyle ou un groupe, éventuellement substitué, alkyle, alcoxy, arylalkyle, alcoxycarbonyle ou alcoxycarbonylalkyle, ou un groupe amino de formule $NR_6R_7$; $R_6$ et $R_7$, qui peuvent être identiques ou différents, représentent chacun un atome d'hydrogène ou un groupe alkyle, aryle ou acyle éventuellement substitué; et X, tant dans la formule I que dans les définitions des substituants de cette formule, représente un atome d'oxygène ou de soufre.

2. Compositions selon la revendication 1, dans lesquelles la substance active est un composé de formule générale I, dans laquelle X représente un atome d'oxygène; A représente un atome d'oxygène, un groupe carbonyle, le groupe $C(R_4)_2$ dans lequel l'un des groupes $R_4$ est un atome d'hydrogène, un groupe alkyle ou alcoxy ayant jusqu'à 4 atomes de carbone, ou un groupe aryle, et l'autre groupe $R_4$ est un atome d'hydrogène ou d'halogène, un groupe hydroxyle, un groupe alkyle ayant jusqu'à 4 atomes de carbone, un groupe alcoxy ayant jusqu'à 4 atomes de carbone, éventuellement substitué par un atome d'halogène, ou un groupe hydroxyle ou alcoxy, un groupe cycloalcoxy de 5 à 8 atomes de carbone, un groupe alcényloxy ou alcynyloxy ayant jusqu'à 6 atomes de carbone, un groupe alcoxycarbonylalcoxy, un groupe alkylthio ayant jusqu'à 4 atomes de carbone, un groupe alcanoyle, un groupe pyridyle, ou un groupe amino de formule $NR_6R_7$, dans laquelle $R_6$ représente un atome d'hydrogène ou un groupe alkyle ayant jusqu'à 4 atomes de carbone et $R_7$ représente un groupe alkyle ayant jusqu'à 4 atomes de carbone, un groupe phényle éventuellement substitué par au moins un atome d'halogène, ou un groupe alkylcarbonyle, alkylcarbamoyle ou alkylsulfonyle; et B représente un atome d'oxygène, le groupe $C(R_4)_2$, dans lequel chacun des groupes $R_4$, qui peuvent être identiques ou différents, représente indépendamment un atome

d'hydrogène ou un groupe alkyle ou alcoxy ayant jusqu'à 4 atomes de carbone, ou le groupe $NR_5$, dans lequel $R_5$ représente un atome d'hydrogène, un groupe alkyle ayant jusqu'à 4 atomes de carbone, un groupe benzyle, ou un groupe alcoxycarbonylalkyle ayant jusqu'à 8 atomes de carbone.

3. Compositions selon la revendication 2, dans lesquelles B représente un atome d'oxygène et A représente le groupe $C(R_4)_2$, dans lequel $R_4$ est tel que défini dans la revendication 2.

4. Compositions selon l'une quelconque des revendications 1, 2 et 3, dans lesquelles $R_1$ représente un groupe trifluorométhyle, $R_2$ représente un atome d'halogène et $R_3$ représente un atome d'hydrogène.

5. Composition selon l'une quelconque des revendications précédentes, qui comprend au moins deux véhicules dont l'un au moins est un agent tensio-actif.

6. Dérivé d'éther de diphényle de formule I selon la revendication 1, dans lequel les substituants ont les significations indiquées dans la revendication 1, à l'exception de l'anhydride 4-(2-chloro-4-trifluorométhyl-phénoxy)phtalique et des composés dans lesquels $R_1$ est un atome d'hydrogène ou un groupe méthyle, $R_2$ et $R_3$ représentent chacun un atome d'hydrogène, A représente

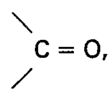

X représente un atome d'oxygène et B représente

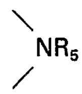

(où $R_5$ est un groupe méthyle ou phényle),

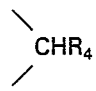

(où $R_4$ est un groupe phényle) ou —O—.

7. Ether de diphényle selon la revendication 6, dans lequel les substituants de la formule I ont les significations indiquées dans l'une quelconque des revendications 2 à 4.

8. Procédé de préparation d'un éther de diphényle selon la revendication 6, qui comprend la réaction d'un phtalide de formule

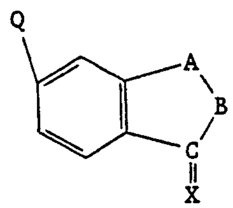

(II)

avec un composé de formule:

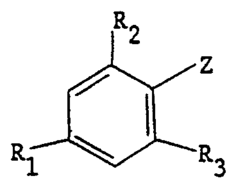

(III)

dans laquelle A, B, $R_1$, $R_2$ et $R_3$ ont les significations indiquées dans la revendication 6; et l'un de Z et Q représente un atome d'halogène ou un groupe nitro et l'autre représente un groupe de formule —OM, dans laquelle M représente un atome d'hydrogène ou de métal alcalin, suivie, lorsqu'on recherche le thiophtalide, de la réaction avec du pentasulfure de phosphore, ou, lorsque le composé recherché est un groupe halogéno ou hydroxyméthylène, de la réaction avec un agent d'halogénation et, pour le dérivé hydroxyméthylène, de l'hydrolyse du dérivé halogénométhylène résultant.

9. Procédé selon la revendication 8, dans lequel la réaction initiale est réalisée par réaction d'un composé de formule II, dans laquelle Q est un groupe hydroxy, avec un hydroxyde de métal alcalin, dans un solvant de type alcanol, suivie de la réaction de l'alcoxyde résultant avec un composé de formule III, dans laquelle Z représente un atome de chlore.

10. Procédé selon la revendication 9, dans lequel la réaction de l'alcoxyde avec le composé chloré III est réalisée dans un solvant organique à une température élevée et sous une atmosphère inerte.

11. Procédé de lutte contre la croissance indésirable des végétaux en un lieu, qui comprend le traitement du lieu avec une composition selon l'une quelconque des revendications 1 à 5 ou avec un composé selon la revendication 6 ou 7.

12. Utilisation comme herbicide d'un éther de diphényle selon l'une quelconque des revendications 1 à 7.